# EUROPÄISCHE PATENTSCHRIFT

(11) **EP 1 651 399 B1**
(45) Veröffentlichungstag und Bekanntmachung des Hinweises auf die Patenterteilung: **16.09.2009**
(21) Anmeldenummer: 04739348.3
(22) Anmeldetag: 26.05.2004
(51) Int. Cl.: B26F 1/38

(54) **VERFAHREN ZUM HERSTELLEN VON HYGIENEARTIKELN AUF VLIESBASIS**
METHOD FOR PRODUCING NONWOVEN-BASED SANITARY ARTICLES
PROCEDE POUR FABRIQUER DES ARTICLES HYGIENIQUES A BASE DE NON-TISSE

(30) Priorität: 25.07.2003 DE 10333942
(43) Veröffentlichungstag der Anmeldung: 03.05.2006
(73) Patentinhaber: Paul Hartmann AG, 89522 Heidenheim (DE)
(72) Erfinder: JUNGE, Michael, 89547 Gerstetten (DE); MANGOLD, Rainer, 89542 Herbrechtingen (DE); LOPIC, Michael, 89278 Nersingen (DE)
(74) Vertreter: Dreiss
(86) Internationale Anmeldenummer: PCT/EP2004/005636
(87) Internationale Veröffentlichungsnummer: WO 2005/018888

(56) Entgegenhaltungen:
- EP-A- 0 679 482
- EP-A- 0 761 397
- DE-A- 3 326 816
- US-A- 4 274 318
- US-A- 5 040 442
- US-A- 5 088 368
- US-A- 5 100 270

## Beschreibung

Die Erfindung betrifft ein Verfahren zum Herstellen von Hygieneartikeln auf Vliesbasis in Form kosmetischer Wattepads, wobei eine zuvor gebildete Vlies- oder Verbundvlieslage einer Schneidstation zugeführt wird, wo eine Vielzahl von Hygieneartikeln aus der Vlies- oder Verbundvlieslage herausgebildet wird.

Bei vorstehend erwähnten Hygieneartikeln handelt es sich um kosmetische Wattepads zum Reinigen der Haut und zum Abschminken, aber auch zum Auftragen kosmetischer oder medizinischer Produkte, wie Cremes, auf die Haut.

Zur Herstellung solcher Hygieneartikel auf Vliesbasis wird üblicherweise eine Vlies- oder Verbundvlieslage hergestellt, aus der dann die Hygieneartikel herausgebildet werden. Unter Vlies bzw. im englischen Sprachgebrauch Nonwoven wird ein unter fachüblichen Vliesbildeverfahren hergestellter Faserverbund entsprechend EDANA Norm 0.0-89, aus dem Jahre 2002, entsprechend der ISO 9092-EN 29092 verstanden. Gewebte, gestrickte, gewirkte und andere textile Stoffe sind somit keine Vliese bzw. Nonwoven.

Bislang werden die Hygieneartikel entweder entsprechend DE 30 22 369 A1 aus der Vlieslage oder Verbundvlieslage herausgestanzt, oder sie werden mittels rotierender Schneidwalzen gemäß EP 0 863 100 A1 aus der Vlieslage herausgetrennt. Bei beiden Herstellverfahren wird eine in der Regel endlose Vlies- oder Verbundvlieslage einer Schneidstation zugeführt, wo dann die Bildung der Hygieneartikel erfolgt. Dabei wird nach dem ersten Verfahren mittels einer Stanzvorrichtung mit einem Formwerkzeug, welches eine bestimmte Anzahl von neben- und/oder hintereinander vorgesehenen Stanzformen einer bestimmten Form und Anordnung aufweist, aus der einlagigen Vliesbahn eine der Anzahl der Stanzformen entsprechende Anzahl von Hygieneartikeln je Stanzhub bildet. Der Wechsel von einer Produktform auf eine andere ist nur durch einen aufwendigen und mit hohen Kosten verbundenen Wechsel des Formwerkzeugs der Stanzvorrichtung möglich. Jedes Produkt erfordert also ein dieser Produktform entsprechendes Formwerkzeug für die Stanzvorrichtung. Hierbei spielt auch die von Anlage zu Anlage variierende Arbeitsbreite der Stanzvorrichtungen eine Rolle. Vorzugsweise sollte die zuvor gebildete Vlies- oder Verbundvlieslage oder -bahn möglichst optimal an die Arbeitsbreite der Stanzvorrichtung angepasst sein, um den Schnittanfall zu minimieren und eine weitgehend optimale Ausnutzung des durch die Vlies- oder Verbundvlieslage zur Verfügung gestellten Materials zur Herstellung der Hygieneartikel zu gewährleisten. Üblicherweise werden Vlies- oder Verbundvliesbahnen mit einer großen Breite von über 2000mm gelegt, so dass diese noch vor dem Aufrollen entsprechend der jeweiligen Arbeitsbreite der betrachteten Stanzvorrichtung geteilt werden müssen. Aufgrund variierender Arbeitsbreiten der Stanzvorrichtungen ist es aber kaum möglich, die volle Breite der gelegten Vliesbahn auszunutzen.

Der im Zuge der Herstellung der Hygieneartikel entstehende Schnittabfall, und zwar sowohl der sogenannte Stanzgitterabfall als auch der Randabfall (Ausschuss infolge nicht erreichter Qualitätsanforderungen, insbesondere hinsichtlich der Vliesdicke im Randbereich), werden dem Vlieslegeprozess bei der Vliesherstellung wieder zugeführt.

Der daraus resultierende Recyclinganteil kann zwischen 30 und 40% betragen. Dieser verhältnismäßig hohe Recyclinganteil kann zu Qualitätsproblemen des Vlieses führen, insbesondere, wenn thermische Verfestigungsverfahren vor dem Schnittvorgang zum Einsatz kommen.

Bei der Ausführung des Stanzvorgangs werden häufig am Schnittrand des Hygieneartikels die Schnittkanten von Ober- und Unterseite miteinander verprägt, so dass die Hygieneartikel in Umfangsrichtung am Schnittrand teilweise oder weitgehend geschlossene Schnittkanten aufweisen, indem Ober- und Unterseite des Hygieneartikels an der Schnittkanten miteinander dauerhaft verbunden sind. Dies resultiert in harten Produkträndern und verhindert ein gleichmäßiges Erscheinungsbild der fertigen Hygieneartikel, was in ästhetischer Hinsicht störend wirkt und außerdem auch die gleichmäßige Beschaffenheit, insbesondere Vliesdicke und - dichte, gefährdet.

Aus DE-A-3 326 816, ist ein verfahren zum schneider eines stapels aus mehreren Flachmateriallagen bekannt.

Der vorliegenden Erfindung liegt die Aufgabe zugrunde, das Verfahren zum Herstellen von Hygieneartikeln auf Vliesbasis in Form kosmetischer Wattepads dahingehend zu verbessern, dass es wirtschaftlicher durchführbar ist, also insbesondere eine größere Anzahl von Hygieneartikeln pro Zeit oder pro Arbeitsvorgang einer Schneidvorrichtung hergestellt werden kann und ferner ein Wechsel der Produktform auf wirtschaftlichere Weise ausführbar ist, und dass eine Verprägung der Schnittränder der Hygieneartikel vermieden wird, die Produktqualität also optimiert werden kann.

Diese Aufgabe wird durch ein Verfahren mit den Merkmalen des Anspruch 1 gelöst.

Mit der vorliegenden Erfindung wurde erkannt, dass durch eine Abkehr vom üblichen Vorgang des Ausstanzens der Hygieneartikel aus einer einlagigen Vliesbahn die Anzahl der pro Zeit oder pro Arbeitsvorgang einer Schneidvorrichtung herstellbaren Hygieneartikel auch dann auf ein wirtschaftliches Maß erhöht werden kann, wenn stattdessen eine mehrlagige Vliesbahn, also eine stapelförmige Anordnung von Vlies- oder Verbundvlieslagen, unter Ausführung eines Schnittvorgangs mittels eines, Schneidmessers in Umfangsrichtung der herauszubildenden Hygieneartikel ausgeführt wird. Dieser Schnittvorgang erfordert, wie vorstehend ausgeführt, die Bewegbarkeit von Schneidmesser und stapelförmiger Anordnung relativ zueinander in Ebenenrichtung der Vlies- oder Verbundvlieslagen und die relative Schwenkbarkeit des Schneidmessers um die erwähnte Achse. Es dürfte sich als am einfachsten ausführbar und realisierbar erweisen, wenn ein Schneidmesser, das insbesondere zusätzlich in z-Richtung hin- und hergehend ausgebildet ist, an einem Schneidkopf der Schneidvorrichtung vorgesehen ist, wobei sich der Schneidkopf dann seinerseits in Ebenenrichtung (x-, y-Richtung) bezüglich einer feststehenden Unterlage und damit bezüglich des Stapels verfahren lässt und gleichzeitig um die z-Richtung verschwenken lässt. Hierdurch sind beliebige. Schnittformen bis zu einem minimalen Radius von etwa 20 mm ausführbar.

Das erfindungsgemäße Verfahren erweist sich insbesondere als vorteilhaft, da eine große Anzahl von übereinander angeordneten Lagen, wenigstens 20, vorzugsweise wenigstens 30, besonders bevorzugt wenigstens 50 und insbesondere bis zu 100 Lagen, in einem Schnittvorgang geschnitten und eine dementsprechende Anzahl von Hygieneartikeln aus dem Stapel bzw. aus den Vlies- oder Verbundvlieslagen herausgebildet werden können. Ferner erweist sich der Übergang von einer Produktform zu einer anderen als ohne weiteres ausführbar, da keine neuen Stanzformen hergestellt, vorgehalten und in die Stanzvorrichtung eingebaut werden müssen, sondern die neue Produktform durch entsprechende Programmierung der Steuervorrichtung der Schneidvorrichtung realisiert werden kann. Es zeigt sich auch, dass aufgrund einer größeren Flexibilität die Breite der zuvor gebildeten Vliesbahn besser ausgenutzt werden kann, indem die Vliesbahn nicht auf die Arbeitsbreite einer Stanzvorrichtung und deren Werkzeug geschnitten werden muss. Hierdurch kann der Faserrecyclinganteil beim Vlieslegeprozess reduziert werden. Ferner ist eine Reduzierung des Stanzgitterabfalls möglich; es kann unter optimaler, insbesondere rechnergestützter Planung des Schneidvorgangs Schnitt an Schnitt geführt und damit ein sehr hoher Ausnutzungsgrad des zur Verfügung stehenden Vlieses je nach Produktform von über 95% erreicht werden.

Ferner zeigt es sich, dass bei der erfindungsgemäßen Herstellung der Hygieneartikel geschlossene Schnittkanten entlang des gesamten Schnittrands der Hygieneartikel vermieden werden können. Dies ist schon beim einlagigen Ausstanzen kaum möglich, es wäre aber beim Stanzen durch eine mehrlagige Vliesbahn völlig ausgeschlossen, weil die Schichten miteinander verprägt werden.

Ein weiterer Vorteil des erfindungsgemäßen Verfahrens ist darin zu sehen, dass bei Vlies- oder Verbundvlieslagen mit unterschiedlichen Oberflächen die Vlieslagen so gelegt werden können, dass jeweils gleiche Oberflächen einander zugewandt sind ("face to face"). Dies kann bei bestimmten Produkten wünschenswert sein.

Es erweist sich des Weiteren als vorteilhaft, dass unter Ausführung des erfindungsgemäßen Verfahrens Hygieneartikel herstellbar sind, die mit hoher Genauigkeit eine. Kennzeichnung, insbesondere eine Prägung, in einem ganz bestimmten stets gleichen Bereich des fertigen Hygieneartikels aufweisen. Hierbei werden üblicherweise Vliesbahnen mit einer geprägten Kennzeichnung oder einer Information im weitesten Sinne verwandt. Beispielsweise durch eine optoelektronische Erfassung der Position solcher geprägter Marken oder solcher Informationen im weitesten Sinne ist es möglich, diese Prägung oder zuvor auf die Vliesbahn aufgebrachte Informationen im weitesten Sinne als Positionsmarke zu erfassen und auszuwerten, um dann den Schneidvorgang so zu steuern, dass diese Prägung oder Information im weitesten Sinne stets an derselben Stelle des Hygieneartikels angeordnet ist.

Die vorstehend erwähnten Vorteile lassen sich also dadurch erreichen, dass anstelle eines Stanzvorgangs oder eines dem Stanzvorgang ähnlichen Herausbildens mittels einer rotierenden Schneidwalze mit in Umfangsrichtung abrollenden Schneidkanten ein Schneidevorgang entlang des gesamten Umfangsrands des herauszubildenden Hygieneartikels unter Ausführung einer translatorischen und einer Drehbewegung durchgeführt wird.

Erfindungsgemäß wird vorgeschlagen, dass der Stapel mehrerer übereinander abgelegter Vlies- oder Verbundvlieslagen oder Abschnitte oder Faltungen von Vlies- oder Verbundvlieslagen komprimiert und im komprimierten Zustand geschnitten wird. Auf diese Weise lässt sich der Schneidvorgang, insbesondere die Qualität der Schnittflächen, verbessern. Außerdem resultiert eine höhere Wirtschaftlichkeit des Verfahrens, da pro Schneidvorgang mehr Produkte erzeugt werden. Eine solche Komprimierung erweist sich bei der Herstellung voluminöser bauschelastischer Hygieneartikel, wie z.B. Wattepads, als zweckmäßig. Es erweist sich als vorteilhaft den Stapel übereinandergelegter Vlieslagen um wenigstens 10% und bis zu 50%, bevorzugt um bis zu 60%, besonders bevorzugt um bis zu 70%, insbesondere um bis zu 80% und ganz besonders bevorzugt um bis zu 90 % der Höhe des Stapels von vorzugsweise wenigstens 30 ohne zusätzliche Belastung übereinandergelegter Vlieslagen zu komprimieren. Zur Berechnung der Komprimierung wird hierbei zunächst die Höhe H1 des Stapels übereinandergelegter Vlieslagen ohne über das Eigengewicht der Lagen hinausgehenden zusätzlichen Prüfdruck ermittelt. Nachfolgend wird die Differenz zwischen dieser Höhe H1 und der Höhe H2, also H1-H2 ins Verhältnis V in % zur Höhe H1 gesetzt, also V = ((H1-H2)/H1)·100, wobei die Höhe H2 die Höhe des Stapels übereinandergelegter Vlieslagen nach der erfolgten Komprimierung ist.

Erfindungsgemäß wird ein Flächengewicht der Einzellagen von 100 bis 400 g/m², insbesondere 150-300 g/m², verwendet.

Der Stapel wird zumindest während des Schneidvorgangs unter der komprimierenden Kraft gehalten. Dies könnte beispielsweise durch Halteleisten, Stempel oder Schablonenelemente erreicht werden. Der Stapel könnte aber auch bereits vor der Ausführung des eigentlichen Schneidvorgangs komprimiert werden, wobei es sich als vorteilhaft erweist, wenn der komprimierte Zustand, so weit wie möglich, für die Ausführung des Schneidvorgangs und vorzugsweise auch danach aufrechterhalten wird.

Für das Komprimieren des Stapels erweist sich insbesondere die Anwendung von Unterdruck, und zwar vorzugsweise unterseitig auf der Auflageseite des Stapels, als vorteilhaft. Die Unterdruckbeaufschlagung kann nämlich ohne weiteres auch während des Schneidvorgangs aufrechterhalten werden, indem der Stapel in der Schneidstation unterseitig mit Unterdruck beaufschlagt wird.

Insbesondere beim Komprimieren des Stapels mittels Unterdruckbeaufschlagung erweist es sich als vorteilhaft, wenn der Stapel beim Komprimieren oder unmittelbar daran anschließend von einer luftundurchlässigen Folienlage überfangen wird. Auf diese Weise lässt sich erreichen, dass der komprimierte Zustand des Stapels entweder nur für verhältnismäßig kurze Zeit, also beispielsweise nur für einen sich unmittelbar daran anschließenden Schneidvorgang, oder für längere Zeit, also beispielsweise zum Zwischenspeichern eines komprimierten Vlieslagenstapels, aufrechterhalten wird. Im letzten Fall würde es sich als vorteilhaft erweisen, wenn der Stapel von der luftundurchlässigen Folienlage möglichst allseitig eingeschlagen ist.

In Weiterbildung dieses Erfindungsgedankens erweist es sich als vorteilhaft, wenn im wesentlichen unmittelbar an das Schneiden eines Stapels oder eines Abschnitts oder Bereichs eines Stapels anschließend der Stapel auf der Eintauchseite des Schneidmessers mit einer luftundurchlässigen Abdeckung, insbesondere einer luftundurchlässigen flexiblen Folienlage, überfangen wird. Auf diese Weise kann nämlich erreicht werden, dass der Unterdruck und damit die Komprimierung des Stapels weitgehend erhalten bleibt.

Es kann sich auch als vorteilhaft erweisen, wenn in einer Herstellungsanlage unter gleichzeitiger Verwendung mehrerer Schneidmesser gleichzeitig mehrere säulenförmige Anordnungen von Hygieneartikeln aus insbesondere demselben Stapel herausgeschnitten werden.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform der Erfindung anhand von Darstellungen einer Herstellungsanlage. In der Zeichnung zeigt:
- Figur 1: eine schematische Darstellung einer Anlage zur Herstellung von Hygieneartikeln in Seitenansicht;
- Figur 2: eine schematische Darstellung der Anlage nach Figur 1 in der Draufsicht; und
- Figur 3: eine schematische Darstellung des Lagenstapels in der Schneidstation (ohne Schneidvorrichtung).

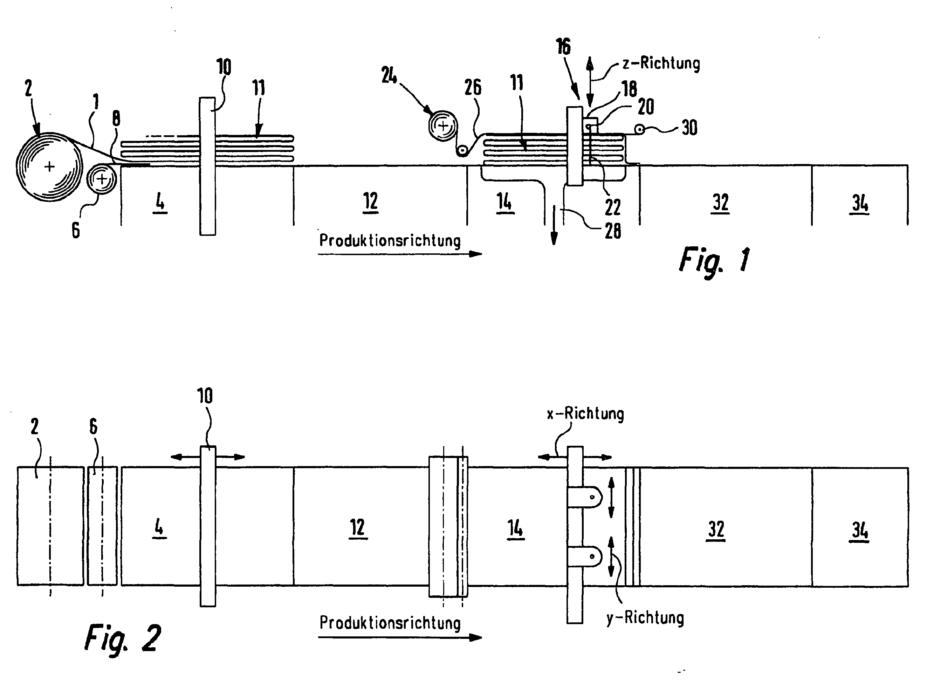

Die Figuren 1 und 2 zeigen in schematischer Darstellung eine Anlage zur Herstellung von Hygieneartikeln auf Vliesbasis, beispielsweise von Wattepads. Hierbei wird eine zuvor hergestellte und aufgerollte Vlies- oder Verbundvliesbahn 1 verwendet. Diese Vlies- oder Verbundvliesbahn 1 wird in einer Abwickeleinrichtung 2 angeordnet, die einem Legetisch 4 vorgeordnet ist. Ebenfalls dem Legetisch 4 vorgeordnet ist eine weitere Abwickeleinrichtung 6 für eine luftdurchlässige Trennlage 8 in Form einer endlosen Papierbahn. Im Bereich des Legetischs 4 der Anlage ist ein schematisch dargestellter Legewagen 10 vorgesehen, mit dem die von der Abwickeleinrichtung 2 abgewickelte Vliesbahn 1 in mehreren, insbesondere wenigstens 30 Lagen, auf dem Legetisch 4 zu einem Stapel 11 gelegt wird. Von dort wird der fertige Stapel 11 zu einem Puffertisch 12 verbracht, wo eine Zwischenlagerung durchführbar ist, um unterschiedliche Geschwindigkeiten bei der Bildung des Stapels und beim anschließenden Schneidvorgang ausgleichen zu können.

In Produktionsrichtung folgt dem Puffertisch 12 eine Schneidstation 14 im weitesten Sinne. Dort ist eine Schneidvorrichtung 16 mit einer Schneidbrücke 18 vorgesehen. Die Schneidbrücke 18 lässt sich in Produktionsrichtung (x-Richtung) hin- und herverfahren. Sie trägt im dargestellten Fall zwei Schneidköpfe 20, die ihrerseits gesteuert quer zur Produktionsrichtung (y-Richtung), also entlang der Schneidbrücke 18, verfahrbar sind. Die Schneidköpfe 20 tragen ein schematisch dargestelltes Schneidmesser 22, welches im wesentlichen senkrecht zur Ebene der Vlieslagen, also zur x-, y-Ebene, erstreckt ist und in dieser Richtung hin- und hergehend entsprechend der Bewegung einer Stichsäge, antreibbar ist. Es wird darauf hingewiesen, dass unabhängig von der sonstigen Ausbildung der Anlage auch mehr als zwei Schneidköpfe vorgesehen sein können. Die zwei oder mehreren Schneidköpfe können unabhängig voneinander verfahrbar sein, oder sie können insbesondere gleichzeitig zur Ausführung derselben Schnittbewegung ansteuerbar sein. Es können auch mehrere Schneidbrücken vorgesehen sein. Hierdurch kann die Anzahl der pro Zeit und insbesondere gleichzeitig herstellbaren Hygieneartikel und die Ausnutzung der Fläche des Stapels optimiert werden.

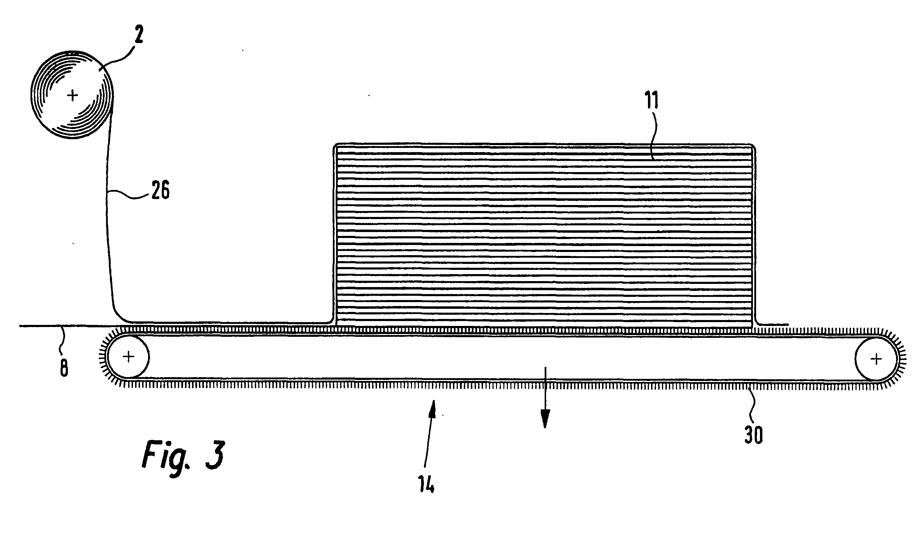

Ferner dargestellt ist eine weitere Abwickeleinrichtung 24 für eine luftundurchlässige Folienbahn 26, welche den in den Figuren 1 und 3 dargestellten Stapel 11 überfängt, was in den Figuren ebenfalls dargestellt ist. Die Aufgabe dieser Folienlage 26 ist es, einen im wesentlichen luftdichten Abschluss des Stapels 11 nach oben und vorzugsweise auch zu den Seiten hin zu bilden, damit der Stapel 11 durch Unterdruckbeaufschlagung von seiner Auflageseite her komprimiert werden kann. Hierfür ist eine Unterdruck erzeugende Einrichtung 28 vorgesehen und in Figur 1 schematisch dargestellt. Die Unterdruckbeaufschlagung erfolgt, wie aus Figur 3 ersichtlich ist, durch ein luftdurchlässiges Transportband 30 hindurch, insbesondere in der Form eines Bürstenbands.

Zur Ausführung des erfindungsgemäßen Verfahrens wird der Stapel 11 bei der vorliegenden Ausführungsform in der Schneidstation 14 mit der Folienlage 26 im wesentlichen luftdicht überfangen und unmittelbar daran anschließend mit Unterdruck beaufschlagt. Hierdurch wird der Stapel komprimiert. Ausgehend von wenigstens 30 lose übereinander liegenden Vlieslagen wird hierbei eine Reduzierung der Stapelhöhe bis zu 20%, insbesondere auf bis zu 30% und weiter insbesondere auf bis zu 40%, wenigstens jedoch auf 80% der Ausgangshöhe des Stapels reduziert. In diesem komprimierten Zustand wird der Stapel 11 dann geschnitten, indem unter Ansteuerung der Schneidvorrichtung 16 die Schneidmesser 22 der beiden Schneidköpfe 20 entlang des gesamten Umfangs der jeweils herzustellenden Hygieneartikel eine Schnittbewegung ausführen. Das Schneidmesser 22 wird also ausgehend von einem Anfangspunkt entlang des Umfangs zu diesem Anfangspunkt zurückgeführt. Hierbei kann der Schnitt auch entlang mehrerer Produkte geführt werden, so dass etwa durch eine sinusförmige oder mäandrierende Schnittführung mehrere Produkte herausgeschnitten werden.

Es erweist sich als vorteilhaft, wenn, wo immer dies möglich ist, unmittelbar im Anschluss an einen Schnitt, die Schnittlinie von der Eintauchseite des Schneidmessers her mit einer luftundurchlässigen Abdeckung, insbesondere auf Folienbasis, überfangen wird, um den Unterdruck im Inneren des Stapels weitgehend aufrechtzuerhalten. Insofern erweist es sich auch als vorteilhaft, dass die Unterdruckbeaufschlagung bei der vorliegend beschriebenen Ausführungsform der Anlage während des Schneidvorgangs aufrechterhalten wird. Gleichwohl erweist sich die vorstehend geschilderte Maßnahme als ratsam und zweckmäßig. Daher ist mit dem Bezugszeichen 30 eine Vorrichtung zur Bereitstellung und Applizierung eines solchen Abdeckmittels im weitesten Sinne schematisch angedeutet. Im Anschluss an die Schneidstation 14 schließt sich eine Art Sortierstation oder Pufferstation 32 an, die einen Übergang zu einer Verpackungsstation 34 bildet.

Unter Ausführung des erfindungsgemäßen Verfahrens kann beispielsweise eine Wattevliesbahn eines Flächengewichts von 170 g/m² und einer Dicke von 4,5 mm, gemessen bei 0,5 kPa Prüfdruck zur Herstellung von Wattepads verwendet werden. Es werden Stapel von 30 bis 40 Lagen dieser Vliesbahn gebildet, die lose übereinander gelegt ohne zusätzliche Belastung eine Höhe von etwa 170 bis 180 mm aufweisen. Es wird dann unter Anlegung eines Unterdrucks eine Komprimierung um 70 bis 80 % dieser Stapelhöhe durchgeführt. In diesem Zustand werden die Stapel dann zur Herstellung der einzelnen Wattepads geschnitten.

## Patentansprüche

1. Verfahren zum Herstellen von kosmetischen Wattepads, wobei eine zuvor gebildete Vlies- oder Verbundvlieslage (1) eines Flächengewichts von 100 - 400 g/m² einer Schneidstation (14) zugeführt wird, wo eine Vielzahl von Wattepads aus der Vlies- oder Verbundvlieslage (1) herausgebildet wird, wobei ein Stapel (11) von wenigstens 20 übereinander abgelegter Vlies- oder Verbundvlieslagen (1) oder Abschnitte oder Faltungen von Vlies- oder Verbundvlieslagen gebildet wird und aus dem Stapel (11) eine der Anzahl der Lagen entsprechende Anzahl von Wattepads in einem Schneidvorgang herausgeschnitten wird, wobei der Stapel (11) und ein im wesentlichen senkrecht zur Ebene der Vlies- oder Verbundvlieslagen des Stapels orientiertes Schneidmesser (22) in der Ebene der Vlies- oder Verbundvlieslagen x-, y-Richtung, relativ zueinander translatorisch bewegbar sind und das Schneidmesser (22) relativ zum Stapel (11) um eine im wesentlichen zu dieser Ebene senkrechte Achse, z-Richtung, drehbar ist und der Stapel (11) vor dem Schneiden komprimiert wird und der Schneidvorgang im komprimierten Zustand unter Ausführung der translatorischen Bewegung und der Drehbewegung zwischen Schneidmesser (22) und Stapel (11) ausgeführt wird und entlang des gesamten Schnittrands der Wattepads offene Schnittkanten erhalten werden.

2. Verfahren nach Anspruch 1, **dadurch gekennzeichnet, dass** bei wenigstens 30 ohne zusätzliche Belastung übereinanderliegenden Lagen des Stapels (11) eine Komprimierung um wenigstens 10% und bis zu 50 %, insbesondere um bis zu 60 %, vorzugsweise um bis zu 70 %, besonders bevorzugt um bis zu 80% und ganz besonders bevorzugt um bis zu 90% der Höhe des Stapels (11) ausgeführt wird.

3. Verfahren nach Anspruch 1 oder 2, **dadurch gekennzeichnet, dass** der Stapel (11) mittels Unterdruckbeaufschlagung komprimiert wird.

4. Verfahren nach einem der Ansprüche 2 oder 3, **dadurch gekennzeichnet, dass** der Stapel (11) beim Komprimieren oder unmittelbar daran anschließend von einer luftundurchlässigen Folienlage (26) überfangen wird.

5. Verfahren nach einem der vorstehenden Ansprüche, **dadurch gekennzeichnet, dass** im wesentlichen unmittelbar an das Schneiden eines Stapels (11) oder eines Abschnitts oder Bereichs des Stapels (11) anschließend der Stapel auf der Eintauchseite des Schneidmessers (22) mit einer luftundurchlässigen Abdeckung überfangen wird.

6. Verfahren nach einem der vorstehenden Ansprüche, **dadurch gekennzeichnet, dass** unter Verwendung mehrerer Schneidmesser (22) gleichzeitig mehrere säulenförmige Anordnungen von Wattepads herausgeschnitten werden.

## Claims

1. A method for producing cosmetic pads of cotton wool, wherein a previously formed fleece or compound fleece layer (1) of a basic weight of 100 to 400 g/m² is conducted to a cutting station (14), in which a plurality of cotton wool pads are formed from the fleece or compound fleece layer (1), wherein a stack (11) is formed by at least twenty fleece or compound fleece layers (1) or sections or folds of fleece or compound fleece layers, and a number of cotton wool pads corresponding to the number of layers is cut out of the stack (11) in one cutting process, wherein the stack (11) and a cutting knife (22), which is oriented substantially perpendicularly in relation to the plane of the fleece or compound fleece layers of the stack, are translatorily movable in the plane of the fleece or compound fleece layers relative to each other in the x-, y-directions, and the cutting knife (22) is rotatable in relation to the stack (11) around an axis, which extends substantially perpendicularly to this plane, z-direction, and the stack (11) is compressed prior to being cut and the cutting process is performed in the compressed state by performing the translatory movement and the rotating movement between the cutting knife (22) and the stack (11), and open cutting edges are obtained along the entire edge of the cut in the cotton wool pad.

2. The method in accordance with claim 1, **characterized in that**, in case of at least thirty layers of the stack (11) placed on top of each other, a compression of the height of the stack (11) by at least 10% and up to 50%, in particular by up to 60%, preferably by up to 70%, particularly by up to 80%, and particularly preferred by up to 90%, is performed.

3. The method in accordance with claim 1 or 2, **characterized in that** the stack (11) is compressed by being charged with an underpressure.

4. The method in accordance with one of claims 2 or 3, **characterized in that** in the course of the compression, or immediately thereafter, the stack (11) is enclosed in a foil layer (26) which is impermeable to air.

5. The method in accordance with one of the preceding claims, **characterized in that** substantially immediately following the cutting of a stack (11), or of a section or area of the stack (11), the stack is enclosed on the penetration side of the cutting knife (22) by a cover which is impermeable to air.

6. The method in accordance with one of the preceding claims, **characterized in that** several column-shaped arrangements of cotton wool pads are cut out by employing several cutting knives (22).

## Revendications

1. Procédé pour fabriquer des disques cosmétiques, dans lequel une couche de non-tissé (1) formée au préalable et d'un poids par unité de surface compris entre 100 et 400 g/m² est amenée à une station de découpe (14), où une pluralité de disques est fabriquée à partir de la couche de non-tissé (1), dans lequel une pile (11) d'au moins 20 couches de non-tissé (1) posées les unes au dessus des autres, ou de segments ou de pliages de couches de non-tissé est réalisée, et dans lequel, à partir de cette pile (11), on découpe lors d'un processus de découpe un nombre de disques correspondant au nombre de couches, dans lequel la pile (11) et une lame (22) orientée de façon essentiellement perpendiculaire au plan des couches de non-tissé de la pile sont mobiles l'une vers l'autre dans un mouvement de translation dans le plan des couches de non-tissé (direction x et y), et dans lequel la lame (22) peut être pivotée par rapport à la pile (11) autour d'un axe essentiellement perpendiculaire à ce plan (direction z), la pile (11) étant comprimée avant la découpe, et le processus de découpe à l'état comprimé s'effectuant par le mouvement de translation et de rotation entre la lame (22) et la pile (11), des arêtes de coupe ouvertes étant obtenues sur toute la longueur du bord des disques.

2. Procédé selon la revendication 1, **caractérisé en ce que**, dans le cas d'une pile (11) d'au moins 30 couches posées les unes au-dessus des autres sans charge supplémentaire, on réalise une compression d'au moins 10 % et jusqu'à 50 %, en particulier jusqu'à 60 %, de préférence jusqu'à 70 %, de façon davantage préférée jusqu'à 80 %, de façon préférée entre toutes jusqu'à 90 % de la hauteur de la pile (11).

3. Procédé selon la revendication 1 ou 2, **caractérisé en ce que** la pile (11) est comprimée par dépression.

4. Procédé selon l'une des revendications 2 ou 3, **caractérisé en ce que** la pile (11) lors de la compression ou immédiatement après est recouverte d'une couche hermétique à l'air (26).

5. Procédé selon l'une des revendications précédentes, **caractérisé en ce que**, essentiellement immédiatement après la découpe d'une pile (11) ou d'un segment de la pile (11), la pile est recouverte sur le côté immergé de la lame (22) d'une couche hermétique à l'air.

6. Procédé selon l'une des revendications précédentes, **caractérisé en ce qu'**en utilisant plusieurs lames (22) en même temps, on découpe plusieurs colonnes de disques.
